# EUROPEAN PATENT APPLICATION

(11) **EP 3 241 453 A1**
(43) Date of publication of application: **08.11.2017**
(21) Application number: 17182706.6
(22) Date of filing: 22.07.2017
(51) Int. Cl.: A24F 47/00, H04W 4/00

(54) **CONTROL METHOD AND CONTROL SYSTEM OF ELECTRONIC CIGARETTE**

(30) Priority: 26.07.2016 CN 201610592622
(71) Applicant: Shenzhen First Union Technology Co., Ltd., Shenzhen, Guangdong 518104 (CN)
(72) Inventor: LI, Yonghai, Shenzhen, Guangdong 518104 (CN); XU, Zhongli, Shenzhen, Guangdong 518104 (CN); CHEN, Hongbin, Shenzhen, Guangdong 518104 (CN); SONG, Shengjia, Shenzhen, Guangdong 518104 (CN); LI, Yuqin, Shenzhen, Guangdong 518104 (CN)
(74) Representative: ProI European Patent Attorneys

(57) **Abstract**

The present disclosure relates to a control method of an electronic cigarette. The electronic cigarette includes an atomizing element, a working circuit, and a power supply. The working circuit is electrically connected to the atomizing element. The control method includes the following steps: (a) establishing a wireless communication connection with a mobile device, the mobile device being configured for emitting a control signal; (b) acquiring receiving the control signal; and (c) if receiving the control signal, controlling the working circuit to connect with the power supply; if not receiving the control signal, returning to step (b).

## Description

### TECHNICAL FIELD

The present invention relates to electronic cigarettes, and particularly to a control method and a control system of an electronic cigarette.

### BACKGROUND ART

Electronic cigarette are harmful for children. A typical electronic cigarette includes a mechanic child lock. However, children may learn to unlock the mechanic child lock easily.

What are needed, therefore, are a control method and a control system of an electronic cigarette, which can overcome the above shortcomings.

### SUMMARY

The present disclosure relates to a control method of an electronic cigarette. The electronic cigarette includes an atomizing element, a working circuit, and a power supply. The working circuit is electrically connected to the atomizing element. The control method includes the following steps: (a) establishing a wireless communication connection with a mobile device, the mobile device being configured for emitting a control signal; (b) acquiring the control signal; and (c) if receiving the control signal, controlling the working circuit to connect with the power supply; if not receiving the control signal, returning to step (b).

### BRIEF DESCRIPTION OF THE DRAWINGS

Many aspects of the present disclosure can be better understood with reference to the following drawings. The components in the drawings are not necessarily drawn to scale, the emphasis instead being placed upon clearly illustrating the principles of the present disclosure. Moreover, in the drawings, like reference numerals designate corresponding parts throughout the several views.
FIG. 1 is a control system of an electronic cigarette according to an embodiment, the control system including the electronic cigarette and a mobile device.
FIG. 2 is a cross-sectional view of the electronic cigarette of FIG. 1.
FIG. 3 is a flowchart of a control method of the electronic cigarette of FIG. 1.

### DETAILED DESCRIPTION

It will be appreciated that for simplicity and clarity of illustration, where appropriate, reference numerals have been repeated among the different figures to indicate corresponding or analogous elements. In addition, numerous specific details are set forth in order to provide a thorough understanding of the embodiments described herein. However, it will be understood by those of ordinary skill in the art that the embodiments described herein can be practiced without these specific details. In other instances, methods, procedures and components have not been described in detail so as not to obscure the related relevant feature being described. Also, the description is not to be considered as limiting the scope of the embodiments described herein. The drawings are not necessarily to scale and the proportions of certain parts have been exaggerated to better illustrate details and features of the present disclosure.

The disclosure is illustrated by way of example and not by way of limitation in the figures of the accompanying drawings in which like references indicate similar elements. It should be noted that references to "an" or "one" embodiment in this disclosure are not necessarily to the same embodiment, and such references mean at least one.

Several definitions that apply throughout this disclosure will now be presented.

The term "outside" refers to a region that is beyond the outermost confines of a physical object. The term "inside" indicates that at least a portion of a region is partially contained within a boundary formed by the object. The term "substantially" is defined to be essentially conforming to the particular dimension, shape or other word that substantially modifies, such that the component need not be exact. For example, substantially cylindrical means that the object resembles a cylinder, but can have one or more deviations from a true cylinder. The term "comprising," when utilized, means "including, but not necessarily limited to"; it specifically indicates open-ended inclusion or membership in the so-described combination, group, series and the like.

The present disclosure relates to a control method of an electronic cigarette 10. The control method includes the following steps:

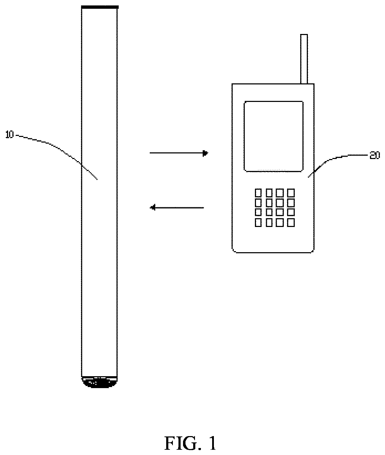

Referring to FIG. 1, a wireless communication between the electronic cigarette 10 and a mobile device 20 is established.

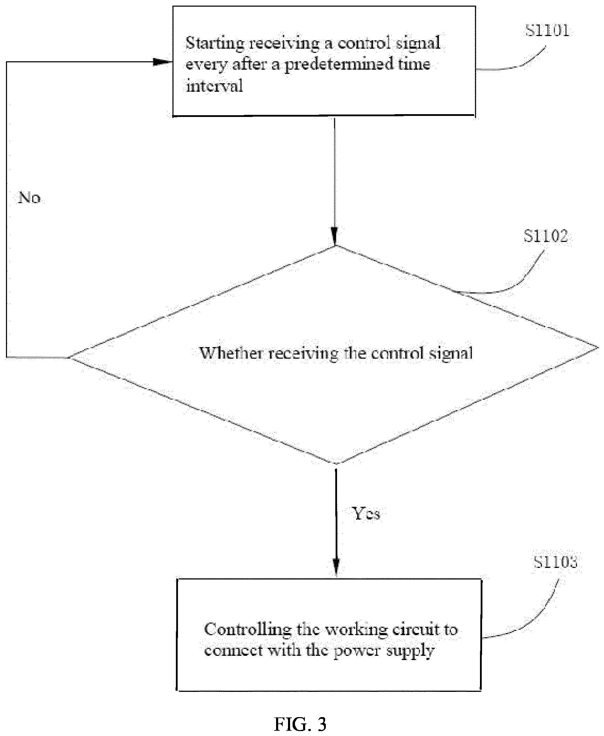

Referring to FIG. 3, in step S1101, the electronic cigarette 10 acquires a control signal emitted by the mobile device 20 every after a predetermined time interval.

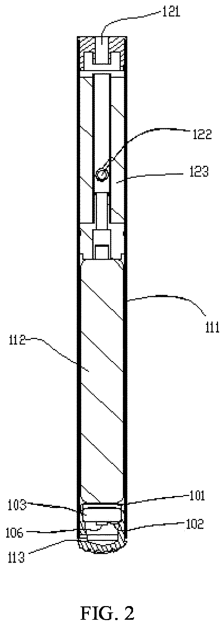

In step S1102, the electronic cigarette 10 judges whether receiving the control signal. If the electronic cigarette 10 receives the control signal, step S1103 is executed; if the electronic cigarette 10 does not receive the control signal, the electronic cigarette 10 returns to step S1102. In step S1103, the electronic cigarette 10 controls an atomizing element 122 to connect to a power supply based on the control signal (see FIG. 2). The atomizing element 122 is configured for atomizing tobacco liquid to form aerosol. In the present embodiment, the predetermined time interval is about 0.2 seconds.

In the present embodiment, when the electronic cigarette 10 receives the control signal, the electronic cigarette 10 controls the working circuit 101 to connect with the power supply.

In the present embodiment, the wireless communication connection between the electronic cigarette 10 and the mobile device 20 may be Bluetooth, infrared, Near Field Communication (NFC), or Wi-Fi. The mobile device 20 may be a cell phone, or a laptop computer.

Referring to FIG. 2, in the present embodiment, the working circuit 101 further includes a switch 103, and the switch 103 is an electronic air actuating switch. The switch 103 is configured for enabling or disenabling the atomizing element 122.

Referring to FIG. 1, a control system of an electronic cigarette is shown. The control system includes the mobile device 20 and the electronic cigarette 10. Referring to FIG. 2, the electronic cigarette 10 includes a housing 111, a power supply 112, a control unit 106, tobacco liquid 123, an atomizing element 122, a working circuit 101, and a switch 103. The power supply 112, the control unit 106, the tobacco liquid 123, the atomizing element 122, the working circuit 101, and the switch 103 are received in the housing 111. The atomizing element 122 is configured for atomizing tobacco liquid to form aerosol. The atomizing element 122 is electrically connected to the working circuit 101. The switch 103 is configured (i.e., structured and arranged) for activating the atomizing element 122 when the working circuit 101 is connected to the power supply 112.

Referring to FIG. 2, an air inlet 113 and an air outlet 121 are defined at two ends of the housing 111. The control unit 106 is configured for controlling the working circuit 101 to connect the power supply 112. The control unit 106 includes a wireless communication element 102. The mobile device 20 is configured for communicating with the wireless communication element 102, and sending a wireless signal to the wireless communication element 102. Based on the wireless signal, the control unit 106 controls the working circuit 101 to connect the power supply 112. In the present embodiment, the switch 103 is an electronic air actuating switch.

In the present embodiment, the wireless communication connection between the electronic cigarette 10 and the mobile device 20 may be Bluetooth, infrared, Near Field Communication (NFC), or Wi-Fi. Correspondingly, the wireless communication element 102 may be a Bluetooth device, an infrared device, an NFC device, or a Wi-Fi device.

The mobile device 20 may be a cell phone, or a laptop computer. When the wireless communication element 102 receives a control signal, the control unit 106 controls the working circuit 101 to connect with the power supply 112.

In the present embodiment, in case a child gets the electronic cigarette 10, he/she needs to get the mobile device 20 if he/she wants to use the electronic cigarette 10. Furthermore, he/she should use the mobile device 20 to emit the control signal for unlocking. Accordingly, it is effective to prevent children from using the electronic cigarette 10.

It is understood that the above-described embodiments are intended to illustrate rather than limit the disclosure. Variations may be made to the embodiments and methods without departing from the spirit of the disclosure. Accordingly, it is appropriate that the appended claims be construed broadly and in a manner consistent with the scope of the disclosure.

## Claims

1. A control method of an electronic cigarette, the electronic cigarette comprising an atomizing element, a working circuit, and a power supply, the working circuit being electrically connected to the atomizing element, the control method comprising:
(a) establishing a wireless communication connection with a mobile device, the mobile device being configured for emitting a control signal;
(b) acquiring the control signal; and
(c) if receiving the control signal, controlling the working circuit to connect with the power supply; if not receiving the control signal, returning to step (b).

2. The control method according to claim 1, wherein the wireless communication connection is achieved by Bluetooth, infrared, NFC, or Wi-Fi.

3. A control system of an electronic cigarette, comprising:
a mobile device;
an electronic cigarette, the electronic cigarette including:
a power supply;
an atomizing element configured for atomizing tobacco liquid;
a control unit, the control unit including a wireless communication element;
a working circuit electrically connecting to the atomizing element,
wherein the mobile device is configured for establishing a wireless communication connection with the wireless communication element, so that the mobile device sends a control signal to the control unit, and the control signal is configured for controlling the working circuit to connect to the power supply.

4. The control system according to claim 3, wherein the control unit is configured for controlling the working circuit to connect to the power supply when the wireless communication element receives the control signal.

5. The control system according to claim 3, wherein the mobile device is a cell phone or a laptop computer.

6. The control system according to claim 3, wherein the wireless communication element is a device selecting from a group consisting of a Bluetooth device, an infrared device, an NFC device and a Wi-Fi device.
